(19) Europäisches Patentamt
European Patent Office
Office européen des brevets

(11) Numéro de publication: **0 101 659**
**B1**

(12) # FASCICULE DE BREVET EUROPEEN

(45) Date de publication du fascicule du brevet: **19.02.86**

(51) Int. Cl.⁴: **C 07 J 41/00**, A 61 K 31/705

(21) Numéro de dépôt: **83401686.7**

(22) Date de dépôt: **19.08.83**

(54) **Nouveaux dérivés d'amino-14 stéroides, procédé pour leur préparation, et application en thérapeutique.**

(30) Priorité: **20.08.82 FR 8214425**
**20.06.83 FR 8310150**

(43) Date de publication de la demande:
**29.02.84 Bulletin 84/9**

(45) Mention de la délivrance du brevet:
**19.02.86 Bulletin 86/8**

(84) Etats contractants désignés:
**AT BE CH DE FR GB IT LI LU NL SE**

(56) Documents cités:
**EP - A - 0 024 983**
**DD - B - 138 983**

**TETRAHEDRON, vol. 34, no. 10, 1978, pages 1487-1491, Pergamon Press Ltd., Oxford, GB, A. ASTIER et al.: "Alcaloides stéroidiques-CLXXVIII: synthèse d'azides tertiaires-VIII synthèse d'azido et d'amino-14beta cardénolides"**

(73) Titulaire: **ETABLISSEMENTS NATIVELLE S.A., 27, rue de la Procession, F-75015 Paris (FR)**

(72) Inventeur: **Jarreau, François-Xavier, 5, rue Louis Hervé, F-78000 Versailles (FR)**
Inventeur: **Koenig, Jean-Jacques, 31, rue du Panorama, F-77672 Vernou la Celle s/Seine (FR)**

(74) Mandataire: **L'Helgoualch, Jean, OFFICE PICARD 134 Boulevard de Clichy, F-75018 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention, réalisée dans les Laboratoires du CERES – Centre Européen de Recherche Scientifique concerne de nouveaux dérivés stéroïdes, et plus particulièrement de nouveaux dérivés d'amino-14 stéroïdes substitués en position 3 par un dérivé sucré, un procédé pour leur préparation et leur application en thérapeutique.

La demande de brevet français N° 2464270 décrit des composés du type amino-14 stéroïdes, et notamment des dérivés hydroxylés de l'amino-14 androstane et de l'amino-14 nor-21 pregnane. On connaît également des alcaloïdes stéroïdiques de la série du pregnane et de landrostane substitués en position 14 par un groupe amino, et par exemple l'amino-14β pregnanediol-3β,20α est décrit par A. Astier *et al.*, «Bull. Soc. Chim.», N° 9-10, pp. 1581-1582 (1976); d'autres amino-14β pregnanes et amino-14β androstanes sont également décrits par A. Astier *et al.*, «Tetrahedron», vol. 34, pp. 1481-1486 (1978). Toutefois aucune propriété pharmacologique ni aucune application thérapeutique de ces dérivés n'a été décrite.

On connaît par ailleurs des dérivés aminés de stéroïdes utiles en thérapeutique, et par exemple les demandes de brevet français N^os^ 2494697 et 2494698 décrivent des amino-3 (5α) pregnanediol-17α,20, des amino-3 (5α) nor-19 pregnanol-20 et des dérivés aminés, présentés comme possédant des propriétés immunothérapeutiques permettant leur application à titre de médicaments pour le traitement des maladies auto-immunes résultant d'une déficience en certains lymphocytes.

Les travaux réalisés par la demanderesse ont permis de constater de manière surprenante que de nouveaux dérivés de stéroïdes aminés, et plus particulièrement des dérivés de la série du pregnanol-20 et du nor-21 pregnanol-20, substitués en position 14 par un groupe amino et en position 3 par un reste sucré, et comportant éventuellement un groupe hydroxyle supplémentaire en position 12, possèdent des propriétés inotropes positives ainsi que des propriétés antiarythmiques supraventriculaires.

La présente invention a donc pour objet de nouveaux dérivés d'amino-14 stéroïdes substitués en position 3 par un dérivé sucré ainsi que leurs sels d'addition avec des acides minéraux ou organiques.

L'invention a également pour objet un procédé de préparation d'amino-14 stéroïdes substitués en position 3 par un dérivé sucré, à partir d'un dihydroxy-3,20 amino-14 stéroïde, ou d'un trihydroxy-3,12,20 amino-14 stéroïde.

L'invention concerne encore l'application de nouveaux dérivés d'amino-14 stéroïdes substitués en position 3 par un dérivé sucré, en thérapeutique humaine et vétérinaire, comme médicaments cardiotoniques pour le traitement de l'insuffisance cardiaque, ainsi que les compositions pharmaceutiques renfermant comme principe actif l'un au moins des nouveaux dérivés d'amino-14 stéroïdes, ou un sel pharmaceutiquement acceptable, en association avec un ou plusieurs excipients appropriés.

Les nouveaux dérivés d'amino-14 stéroïdes conformes à la présente invention peuvent être représentés par la formule générale (I):

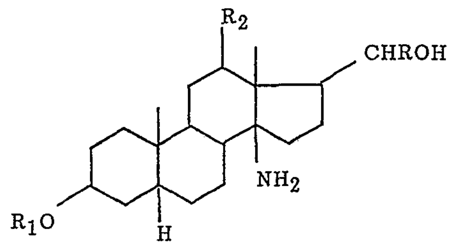

(I)

dans laquelle R représente un atome d'hydrogène ou un groupe alkyle inférieur de 1 à 4 atomes de carbone, $R_1$ représente un reste de sucre, substitué le cas échéant, et $R_2$ représente un atome d'hydrogène, un groupe hydroxyle ou un groupe $-OR_3$, $R_3$ étant un reste de sucre, substitué le cas échéant.

Les nouveaux dérivés d'amino-14 stéroïdes de formule générale (I) comportent dans leur molécule plusieurs atomes de carbone asymétriques, en particulier les carbones en positions 3, 5, 14, 17 et 21, ainsi que le carbone en position 12 lorsque $R_2$ n'est pas un atome d'hydrogène, et peuvent donc exister sous diverses formes stéréo-isomères; l'invention concerne les nouveaux produits de formule générale (I) sous forme d'isomères séparés ou en mélange.

L'invention concerne aussi les sels des amino-14 stéroïdes de formule générale (I) et, en particulier, les sels obtenus par action d'un acide minéral ou organique, suivant les méthodes usuelles de la technique. L'acide utilisé peut être choisi parmi l'acide chlorhydrique, l'acide oxalique, l'acide tartrique, l'acide fumarique, l'acide lactique, l'acide phosphorique, l'acide p-toluène sulfonique, l'acide formique, l'acide bromhydrique, l'acide maléique, l'acide sulfamique, etc.

Dans la formule générale (I) ci-dessus, R représente de préférence un atome d'hydrogène ou un groupe méthyle.

Le reste sucré représenté par $R_1$ dans la formule générale (I) peut être un reste de monosaccharide, d'oligosaccharide ou de polysaccharide, substitué le cas échéant.

$R_1$ peut être par exemple un reste de monosaccharide, et notamment un pentose ou un hexose modifié ou substitué le cas échéant, pour former par exemple un désoxy-2 ou un désoxy-6 hexose, un didésoxy-2,6 hexose, un désoxy-2 amino-2 hexose, un désoxy-3 amino-3 hexose, un désoxy-3 méthoxy-3 hexose, un tridésoxy-2,3,6 hexose, un didésoxy-4,6 méthoxy-4 hexose, un tridésoxy-2,3,6 didéhydro-2,3 hexose, etc.

Comme exemples de monosaccharides utilisables dans l'invention on peut citer notamment le glucose, le rhamnose, le fructose, le galactose, le mannose, l'arabinose, le digitoxose, le cymarose, le xylose, le lyxose, le ribose, le digitalose, le désoxy-6 glucose, la glucosamine, l'amino-4 tridésoxy-2,4,6 lyxohexopyrannose, l'amino-4 didésoxy-4,6 glycopyrannose, le didésoxy-2,3 rham-

nopyrannose, le méthoxy-4 didésoxy-4,6 rhamnopyrannose, etc., et de préférence les anomères β-D ou α-L. On peut également utiliser des disaccharides tels que le saccharose, le maltose ou le lactose ou encore divers polysaccharides contenant plusieurs oses.

Parmi les composés de formule générale (I) ci-dessus, l'invention concerne de préférence ceux pour lesquels le reste sucré représenté par $R_1$ est un reste de glucose, de rhamnose, de galactose, de fucose ou de digitoxose.

Comme indiqué ci-dessus, les dérivés d'amino-14 stéroïdes suivant l'invention peuvent exister sous diverses formes stéréo-isomères résultant de la présence de plusieurs atomes de carbone asymétriques dans le squelette stéroïdique. L'invention concerne de préférence les dérivés de formule générale (I) dans laquelle le substituant $-OR_1$ en position 3, l'atome d'hydrogène en position 5, le groupe $R_2$ en position 12, le groupe $-NH_2$ en position 14, et le substituant $-CHROH$ en position 17 ont la configuration β. Cette configuration n'est pas limitative et par exemple l'atome d'hydrogène en position 5 peut être en configuration α. Le groupe $-OH$ en position 20 peut avoir l'une ou l'autre des configurations α et β quand R est un groupe alkyle.

L'invention concerne tout particulièrement les dérivés d'amino-14 stéroïdes constitués par le O-(α-L-rhamnopyrannosyl)-3 amino-14α nor-21 pregnane-5β diol-3β,20, le O-(α-L-rhamnopyrannosyl)-3 amino-14β pregnane-5β diol-3β,20α et son isomère 20β, le O-(β-D-digitoxosyl)-3 amino-14β pregnane-5β diol-3β,20α, le O-(amino-4 tridésoxy-2,3,6 α-L-lyxohexopyrannosyl)-3 amino-14β pregnane-5β diol-3β,20α, le O-(α-L-rhamnopyrannosyl)-3 amino-14β nor-21 pregnane-5β triol-3β,12β,20, le O-(α-L-rhamnopyrannosyl)-3 amino-14β pregnane-5β triol-3β,12β,20β et son isomère 20α, le O-(β-D-digitoxosyl)-3 amino-14β pregnane-5β triol-3β,12β,20β et son isomère 20α, et le di-O-(α-L-rhamnopyrannosyl)-3,12 amino-14β pregnane-5β triol-3β,12β,20β.

Les nouveaux dérivés d'amino-14 stéroïdes de formule générale (I) peuvent être préparés à partir des amino-14 stéroïdes représentés par la formule générale (II) ci-après:

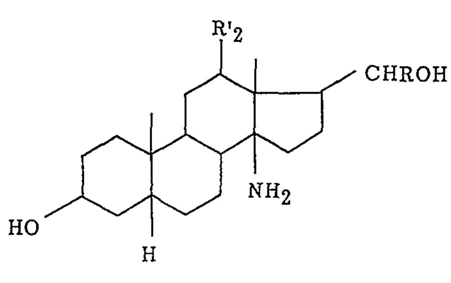

(II)

dans laquelle R est un atome d'hydrogène ou un groupe alkyle inférieur et, de préférence, un groupe méthyle, et $R'_2$ est un atome d'hydrogène ou un groupe hydroxyle, en protégeant le groupe $-NH_2$ en position 14 et le groupe $-OH$ en position 20, ainsi que, le cas échéant, le groupe $-OH$ en position 12 représenté ar $R'_2$, en effectuant une réaction de couplage avec un sucre activé de formule $R_1-X$ où $R_1$ est défini comme ci-dessus et X représente un atome d'halogène ou un groupe acétyle, et en éliminant ensuite, le cas échéant, les groupements protecteurs.

Les sucres activés peuvent être par exemple le tri-O-acétyl digitoxose, le bromure de tri-O-acétyl α-L-rhamnosyle, le O-benzoyl-3 tridésoxy-2,4,6 trifluoroacétamido-4 α,β-L-lyxohexopyrannoside d'acétyle, le bromure de di-O-acétyl-2,3 didésoxy-4,6 trifluoroacétamido-4 glycopyrannosyle, le bromure de tétra-O-acétyl α-D-glycosyle, etc.

La protection du groupe $-NH_2$ en position 14 et du groupe $-OH$ en position 20 s'effectue par les techniques usuelles, et par exemple le groupe $-NH_2$ peut être transformé en groupe trifluoroacétamido $-NHCOCF_3$ par action de l'anhydride d'acide trifluoroacétique en présence de triéthylamine, ou en groupe formamido par action de l'anhydride acétique dans l'acide formique. Le groupe $-OH$ peut être acétylé par action de l'anhydride acétique dans un solvant approprié tel que la pyridine.

Les groupements fonctionnels des dérivés sucrés sont de préférence protégés avant d'effecteur la réaction de couplage avec l'amino-14 stéroïde. Les groupes hydroxyles des dérivés sucrés peuvent être protégés par les méthodes usuelles, notamment par acylation ou par benzoylation. Par exemple les groupes hydroxyle d'un digitoxose ou d'un rhamnose peuvent être transformés en groupe acétoxy par acétylation au moyen de l'anhydride acétique. On pourra notamment se référer à la méthode décrite par E. Fisher *et al.* dans «Chem. Ber.», N° 53, p. 2362 (1920), pour la technique de protection des sucres. Les groupes amino éventuellement présents dans le dérivé sucré sont également préalablement protégés par un groupement acyle, et de préférence par un groupement trifluoroacétyle.

Les groupements protecteurs sont ensuite éliminés, après le couplage entre le dérivé sucré et l'amino-4 stéroïde, suivant les techniques usuelles, par exemple par chauffage en milieu basique.

La réaction de couplage s'effectue en faisant réagir au niveau de son atome de carbone anomère, le dérivé sucré préalablement protégé, avec le groupe hydroxy en position 3 de l'amino-14 stéroïde sur lequel on a introduit des groupements protecteurs au niveau des substituants des positions 14 et 20, dans un solvant organique approprié. On utilise généralement un équivalent de dérivé sucré, mais on peut aussi l'utiliser en excès, et par exemple, on peut utiliser entre 1,5 et 2,5 mol de dérivé sucré pour une mole d'amino-14 stéroïde.

Les conditions opératoires peuvent être choisies suivant les méthodes usuelles, et par exemple la méthode de couplage décrite par D.M. von Niekerk *et al.* dans «Experientia», N° 28, p. 123 (1972), ou encore la méthode décrite par W. Meyer *et al.* dans «Chem. Ber.», N° 104, p. 1 (1971) notamment dans le cas d'un sucre aminé. Lorsque l'on utilise un sucre tel qu'un didésoxy-2,6 hexose ou un amino-4 tridésoxy-2,4,6 hexose, il est avantageux d'effectuer le couplage suivant la

méthode décrite par J. Boivin, C. Monneret et M. Païs dans «Tetrahedron Letters», p. 1111 (1980).

La réaction de couplage peut s'effectuer suivant les techniques ci-dessus, dans un solvant tel que le benzène ou le toluène, l'acétonitrile, le chlorure de méthylène ou le dioxanne, isolément ou en mélange, à température ambiante, mais il peut être utile de chauffer le milieu réactionnel pour activer la réaction, en fonction des réactifs utilisés.

La réaction de couplage s'effectue préférentiellement avec le groupement hydroxyle en position 3 de l'amino-14 stéroïde protégé en 14 et en 20 mais non protégé en 12, la réactivité du groupe hydroxyle en 3 étant plus grande que celle du groupe hydroxyle présent le cas échéant en 12. Le dérivé sucré en 3 et en 12 formé comme produit secondaire au cours de la réaction de couplage est aisément séparé. Pour former de manière prédominante le dérivé sucré en 3 et 12, on peut faire agir le dérivé sucré en excès.

Les amino-14 stéroïdes de formule générale (II) utilisés comme produits de départ, où $R'_2$ est un atome d'hydrogène, peuvent être préparés comme indiqué dans la demande de brevet français N° 82.14038 au nom de la demanderesse, par réduction puis acétylation d'un hydroxy-3,14 stéroïde portant en position 17 un groupement acyle −COR, R étant un atome d'hydrogène ou un groupe alkyle, pour former un trihydroxy-3,14,20 stéroïde O-acétylé en positions 3 et 20, que l'on traite par un complexe acide azothydriquetrifluorure de bore avant d'effectuer une réduction par un hydrure métallique ou par hydrogénation catalytique. De même, les amino-14 stéroïdes formule générale (II) où $R'_2$ est un groupe hydroxyle, peuvent être préparés par le même procédé, à partir d'un trihydroxy-3,12,20 stéroïde, comme indiqué dans la demande de brevet français N° 83.10031 au nom de la demanderesse.

Par exemple, l'amino-14β pregnane-5β diol-3β,20α représenté par la formule (II) où R est un groupe méthyle, et $R'_2$ est un atome d'hydrogène, peut être obtenu à partir de l'oxo-20 pregnane-5β diol-3β,14β sur lequel on effectue une réduction par le borohydrure de potassium, puis une acétylation par l'anhydride acétique dans la pyridine pour former le di-O-acétyl-3,20 pregnane-5β triol-3β,14β,20β que l'on traite par l'acide azothydrique en présence d'éthérate de trifluorure de bore, pour effectuer ensuite une réduction par l'hydrure mixte de lithium et d'aluminium. On peut aussi utiliser la méthode décrite par Astier *et al.* dans «Tetrahedron», N° 34, pp. 1481-1486 (1978).

Comme indiqué ci-dessus, les dérivés d'amino-14 stéroïdes représentés par la formule générale (I) possèdent d'intéressantes propriétés pharmacologiques, notamment des propriétés inotropes positives et des propriétés antiarythmiques supraventriculaires décrites plus en détail ci-après permettant leur application en thérapeutique humaine et vétérinaire pour le traitement de l'insuffisance cardiaque.

Les exemples décrits ci-après illustrent l'invention.

*Exemple 1:*

*O-(β-D-digitoxoxyl)-3 amino-14β nor-21 pregnane-5β diol-3β,20*

A une solution de 3,2 g d'amino-14β nor-21 pregnane-5β diol-3β,20 dans 40 ml de pyridine refroidie à 0° C, on ajoute sous agitation 1 ml d'anhydride acétique.

Après 30 min d'agitation à 0° C, le milieu réactionnel est additionné d'une solution de bicarbonate de sodium glacé, laissé sous agitation pendant encore 10 min, puis extrait par du chlorure de méthylène et évaporé à sec.

Par cristallisation du résidu (3,62 g) dans le mélange benzène/hexane, 3,0 g de O-acétyl-20 amino-14β nor-21 pregnane-5β diol-3β,20 sont obtenus (rendement 83%).

A une solution de 3,66 g du dérivé ci-dessus dans 100 ml de chlorure de méthylène anhydre refroidi à 0° C, on ajoute, sous agitation magnétique, 3,4 ml de triéthylamine et 3,1 ml d'anhydride trifluoroacétique. On agite encore 20 min en laissant revenir à la température ambiante, puis on évapore à sec. Le résidu est dissous à nouveau dans du chlorure de méthylène et la solution obtenue est lavée par une solution saturée de bicarbonate de sodium, puis à l'eau, enfin séchée de façon habituelle et évaporée à sec. Le produit obtenu est dissous dans 600 ml de méthanol, puis passé lentement en l'espace de 5 h sur une colonne de 300 ml de résine IR 45 (phase OH). La colonne est lavée avec 300 ml de méthanol. Les solutions recueillies, évaporées à sec, fournissent 4,52 g de O-acétyl-20 trifluoroacétamido-14β nor-21 pregnane-5β diol-3β,20 pur en chromatographie sur couche mince, qui ne cristallise pas.

A une solution de 0,33 g du dérivé ci-dessus et de 0,40 g de tri-O-acétyl digitoxose dans 30 ml de benzène, on ajoute 0,35 g d'acide p-toluène sulfonique sec. Après 1 h d'agitation à la température ambiante, le milieu réactionnel est additionné d'une solution de bicarbonate de sodium saturée, et extrait par du chlorure de méthylène. Le résidu (0,6 g) est chromatographié sur silice Merck H60 (éluant hexane/AcOEt 75/25). On obtient 0,4 g d'un mélange des O-(di-O-acétyl-3,4α et β -D-digitoxosyl)-3 O-acétyl-20 trifluoracétamido-14β nor-21 pregnane-5β diol-3β,20.

A une solution de ce mélange dans 16 ml de méthanol, on ajoute, sous agitation, 4 ml de soude 5N et on chauffe à reflux pendant 2½ h. Après ce temps, on dilue à l'eau et extrait par du chlorure de méthylène. Le produit obtenu (0,27 g) est chromatographié sur silice Merck H60 (éluant $CH_2Cl_2/MeOH/NH_4OH$ 93/7/0,4). On obtient ainsi 0,15 g du dérivé β-D qui cristallise dans le mélange méthanol/éther, ainsi que 0,11 g de l'anomère α-D, qui cristallise dans le même mélange de solvants.

Point de fusion F = 198-199° C (anomère β-D). 226° C (anomère α-D).

$/\alpha_D/ = -31°$ (c = 1, $CHCl_3$)

Spectre de RMN ($CDCl_3$)

δ = 0,93 ($CH_3$-19) 0,98 ($CH_3$-18) 1,30 ($CH_3$-6') 4,87 (H-1') ppm (anomère β-D).

δ= 0,95 ($CH_3$-19) 1,00 ($CH_3$-18) 1,31 ($CH_3$-6') 4,99 (H-1') ppm (anomère α-D).

*Exemple 2:*

*O-(β-L-rhamnopyrannosyl)-3 amino-14β nor-21 pregnane-5β diol-3β,20*

A une solution contenant 4,6 g d'acétyl-20 trifluoroacétamido-14β nor-21 pregnane-5β diol-3β,20 obtenu comme indiqué dans l'exemple 1, et 6,95 g de bromure de tri-O-acétyl-2,3,4α-L-rhamnosyle dans 235 ml d'acétonitrile, on ajoute 4,96 g de cyanure mercurique. Après 1 h d'agitation à la température ambiante, on dilue par du chlorure de méthylène et extrait en lavant par une solution saturée d'hydrogénocarbonate de sodium. Le produit brut obtenu (7,82 g) est chromatographié sur silice Merck H60 (éluant $CH_2Cl_2$/MeOH 0,3%).

On obtient 5,36 g de O-(tri-O-acétyl-2,3,4 α-L-rhamnopyrannosyl)-3β O-acétyl-20 trifluoroacétamido-14β nor-21 pregnane-5β diol-3β,20 pur qui ne cristallise pas.

A 4,6 g du dérivé ci-dessus en solution dans 130 ml de méthanol, on ajoute 32 ml de soude 5N et on chauffe à reflux pendant 4 h. On dilue ensuite par du chlorure de méthylène et on lave à l'eau, puis, par une solution de chlorure de sodium à demi-saturation (en maintenant une concentration de méthanol suffisante). On obtient 3 g de produit pur en chromatographie sur couche mince, qui cristallise dans le mélange méthanol/éther.

Point de fusion F = 244° C

/$\alpha_D$/ = −53° (c = 1, $CHCl_3$/MeOH 80/20)

Spectre de RMN ($CDCl_3$)

δ = 0,90 ($CH_3$-19) 0,95 ($CH_3$-18) 1,20 ($CH_3$-6') 4,82 (H-1') ppm.

*Exemple 3:*

*O-(amino-4 didésoxy-4,6 β-D-glucopyrannosyl)-3 amino-14β nor-21 pregnane-5β diol-3β,20*

A une solution benzénique contenant 0,6 g de O-acétyl-20 trifluoroacétamido-14β nor-21 pregnane-5β diol-3β,20 obtenu comme indiqué dans l'exemple 1, on ajoute 0,35 g de cyanure mercurique et 0,24 g de bromure mercurique. On porte à reflux et on ajoute 0,27 g de bromure de diacétyl-2,3 didésoxy-4,6 trifluoroacétamido-4 glucopyrannosyle en solution dans 6 ml de benzène. Après 1 h d'ébullition à reflux, on ajoute encore la même quantité du bromosucre (0,27 g dans 6 ml de benzène) et après encore 1 h de nouveau 0,27 g de bromosucre dans 4 ml de benzène. On laisse refluer pendant 2,5 h, puis on refroidit, on dilue par du chlorure de méthylène et on extrait en lavant par une solution saturée de bicarbonate de sodium. Le solvant organique évaporé laisse un résidu (1,05 g) qui est chromatographié sur silice Merck H60 (éluant $CH_2Cl_2$/MeOH 0,4%). On obtient 0,46 g de produit pur en CCM, qui cristallise dans le mélange acétone/hexane.

A une solution de 0,35 g du produit ci-dessus dans 14 ml de méthanol, on ajoute 3,5 ml de soude 5N et on chauffe à reflux pendant 3 h, on dilue ensuite à l'eau et on extrait par du chlorure de méthylène. On obtient 0,18 g de produit qui cristallise dans le mélange éthanol/éther.

Point de fusion F = 233° C

/$\alpha_D$/ = −34° (c = 1, $CHCl_3$).

*Exemple 4:*

*O-(amino-4 tridésoxy-2,4,6 α-L-lyxohexopyrannosyl)-3 amino-14β nor-21 pregnane-5β diol-3β,20*

A une solution de 1,8 g d'amino-14β nor-21 pregnane-5β diol-3β,20 décrit dans l'exemple 6 du brevet français N° 2464270, dans 39,6 ml d'acide formique, on ajoute à température ambiante, 24 ml d'anhydride acétique. On porte la température du milieu à 60° C pendant 30 min, puis à 100° C. On ajoute alors 8 ml d'anhydride acétique et on laisse réagir environ 1 h à la même température.

Après refroidissement et dilution à l'eau, on extrait par le dichlorométhane, on lave par l'eau bicarbonatée, on sèche sur sulfate de sodium et on évapore à sec sous vide.

Il s'est formé 2,17 g d'un mélange des deux produits neutres, auquel on ajoute 42 ml de soude éthanolique 0,25N refroidie à +5° C. Après 1 h à température ordinaire, on dilue par l'eau, on extrait par le chlorure de méthylène et après lavage, on obtient 2,28 g d'un résidu sous forme de mousse (rendement brut quantitatif).

On refroidit à −15° C par un bain de glace-sel, une solution de 2,18 g du produit précédent dans 12 ml de pyridine. On ajoute 0,55 g d'anhydride acétique et on laisse remonter à température ambiante sans enlever le bain, en 2 h; on effectue ensuite deux additions d'anhydride acétique de 0,16 g chaque fois, à −15° C, en laissant remonter la température entre chaque addition.

On ajoute 120 ml d'eau. On extrait par le benzène, on lave par l'acide citrique en solution aqueuse à 5%, puis à l'eau.

Le produit brut ainsi obtenu est chromatographié sur colonne de silice; on utilise comme éluant un mélange dichlorométhane/méthanol (98/2) pour séparer, après cristallisation dans le mélange benzène/éther isopropylique, 1,25 g d'acétyl-20 formylamino-14β nor-21 pregnane-5β diol-3β,20 (rendement 60%).

A une solution de 0,60 g du produit ci-dessus et de 1,16 g de O-benzoyl-3 tridésoxy-2,4,6 trifluoroacétamido-4 α,β-L-lyxohexopyrannoside d'acétyle dans 80 ml d'un mélange à parties égales de benzène et de chlorure de méthylène, on ajoute 0,5 g d'acide p-toluène sulfonique sec et laisse agiter à la température ambiante pendant 3 h, on ajoute ensuite une solution aqueuse saturée de bicarbonate de sodium et extrait par du chlorure de méthylène. Le produit obtenu (1,66 g) est chromatographié sur 50 g de silice Merck H60 (éluant: hexane/acétone 3/1) et fournit 0,84 g d'un mélange des anomères α et β-L. Par cristallisation dans le mélange acétone/hexane, on sépare l'anomère α-L pur, soit 0,50 g.

L'anomère α-L est mis en solution dans 24 ml de méthanol, on ajoute 6 ml de soude 5N et on

chauffe à reflux pendant 1 h. On dilue par de l'eau et on extrait par du chloroforme. On élimine ainsi les groupements protecteurs et on obtient 0,36 g du produit recherché, qui cristallise dans le mélange méthanol/éther.

Point de fusion F = 219-220° C

/$\alpha_D$/ = −91° (c = 1, $CHCl_3$)

Spectre de RMN ($CDCl_3$)

δ = 0,93 ($CH_3$-19) 1,00 ($CH_3$-18) 1,17 ($CH_3$-6') 4,91 (H-1') ppm.

*Exemple 5:*

*O-(β-D-digitoxosyl)-3 amino-14β pregnane-5β diol-3β, 20β*

Suivant le procédé décrit dans l'exemple 1, on traite 1,7 g d'amino-14β pregnane-5β diol-3β,20α par l'anhydride acétique, puis on fait réagir le produit obtenu (non cristallisé) avec l'anhydride trifluoroacétique pour former 2,4 g de O-acétyl-20 trifluoroacétamido-14β pregnane-5β diol-3β,20α qui cristallise dans le mélange éther/hexane.

En procédant comme dans l'exemple 1, on fait réagir 1,1 g de tri-O-acétyl digitoxose sur 0,9 g du produit ci-dessus dans 120 ml de benzène en présence de 0,8 g d'acide p-toluène sulfonique sec.

Après lavage et extraction, puis traitement par la soude en chauffant à reflux, puis purification, on obtient 0,35 g de l'anomère β-D ainsi que 0,26 g de l'anomère α-D qui cristallise tous deux dans le mélange méthanol/éther diéthylique.

Point de fusion F = 208° C (anomère β-D)

/$\alpha_D$/ = −26° (c = 1,5 $CHCl_3$).

*Exemple 6:*

*O-(α-L-rhamnopyrannosyl)-3 amino-14β pregnane-5β diol-3β, 20α*

On procède comme dans l'exemple 5, en remplaçant le tri-O-acétyl digitoxose par le bromure de tri-O-acétyl-2,3,4 α-L-rhamnosyle dans l'acétonitrile, en présence de cyanure mercurique.

Après 1 h de réaction à température ambiante, dilution au chlorure de méthylène et lavage par une solution saturée de bicarbonate de sodium, on obtient le O-(tri-O-acétyl-2,3,4 α-L-rhamnopyrannosyl)-3β O-acétyl-20 trifluoroacétamido-14β pregnane-5β diol-3β,20α que l'on purifie par chromatographie sur colonne de silice.

Le produit ci-dessus, en solution dans le méthanol, est additionné de soude 5N et chauffé à reflux pendant 8 h. Après dilution à l'eau et extraction par du chlorure de méthylène, on obtient le O-(α-L-rhamnopyrannosyl)-3 amino-14β pregnane-5β diol-3β,20α qui cristallise dans le mélange éthanol/éther diéthylique.

Point de fusion F = 265° C

/$\alpha_D$/ = −49° (c = 0,8 $CHCl_3$/MeOH 80/20)

Spectre de RMN ($CDCl_3$ + $CD_4O$)

δ = 0,96 ($CH_3$−19) 1,00 ($CH_3$−18) 1,06 ($CH_3$−21) 1,28 ($CH_3$−6') 4,90 (H−1') ppm.

*Exemple 7:*

*O-(α-L-rhamnopyrannosyl)-3 amino-14β pregnane-5β diol-3β,20β*

En procédant comme dans l'exemple 1, on prépare l'acétoxy-20 trifluoroacétamido-14β pregnane-5β diol-3β,20β en faisant agir l'anhydride acétique, puis l'anhydride trifluoroacétique sur l'amino-14β pregnane-5β diol-3β,20β.

On fait réagir le produit obtenu avec le bromure de tri-O-acétyl-2,3,4 α-L-rhamnosyle dans l'acétonitrile en présence de cyanure mercurique, suivant le mode opératoire de couplage décrit dans l'exemple 2. Après traitement par la soude dans le méthanol en chauffant à reflux, on obtient le O-(α-L-rhamnopyrannosyl)-3 amino-14β pregnane-5β diol-3β,20β qui cristallise dans l'éthanol.

Point de fusion F = 263-264° C

/$\alpha_D$/ = −52° (c = 0,7 $CHCl_3$/MeOH 80/20)

Spectre de RMN ($CDCl_3$ + $CD_4O$)

δ = 0,95 ($CH_3$-19) 1,16 ($CH_3$-18) 1,30 ($CH_3$-21) 1,30 ($CH_3$-6') 4,83 (H-1') ppm.

*Exemple 8:*

*O-(α-L-rhamnopyrannosyl)-3 amino-14β pregnanediol-3β,20α*

On procède comme dans l'exemple 6 en remplaçant l'amino-14β pregnane-5β diol-3β,20α par l'amino-14β pregnanediol-3β,20α isomère dont le proton en position 5 occupe la configuration 5α, et en effectuant le couplage avec le bromure de tri-O-acétyl-2,3,4 α-L-rhamnosyle dans les mêmes conditions.

Après traitement par la soude en solution dans le méthanol, en chauffant à reflux, on obtient le O-(α-L-rhamnopyrannosyl)-3β amino-14β pregnanol-20α qui cristallise dans le mélange éthanol/éther.

Point de fusion F = 271° C

Spectre RMN ($CDCl_3$ + $CD_4O$)

δ = 0,78 ($CH_3$-19) 0,98 ($CH_3$-18) 1,03 ($CH_3$-21) 1,25 ($CH_3$-6') 4,76 (H-1') ppm.

*Exemple 9:*

*O-(amino-4 tridésoxy-2,4,6 α-L-lyxohexopyrannosyl)-3 amino-14β pregnane-5β diol-3β, 20α*

On procède comme dans l'exemple 4 en remplaçant l'amino-14β nor-21 pregnane-5β diol-3β,20 par l'amino-14β pregnane-5β diol-3β,20α pour obtenir le produit recherché qui cristallise dans le mélange méthanol/éther éthylique.

Point de fusion F = 224° C

/$\alpha_D$/ = −79° (c = 1, $CHCl_3$).

*Exemple 10:*

*O-(α-L-rhamnopyrannosyl)-3 amino-14β pregnane-5β triol-3β,12β,20β*

On ajoute 1 ml d'anhydride à une solution de 50 ml de pyridine refroidie à 0° C contenant 3,5 g d'amino-14β pregnane-5β triol-3β,12β,20β en maintenant le mélange réactionnel sous agitation. On laisse réagir pendant 30 min environ à 0° C sous agitation, puis on ajoute une solution de bicarbonate de sodium glacé. Après 10 min, on extrait par du chlorure de méthylène et on élimine le solvant par évaporation.

On obtient ainsi 3,7 g d'O-acétyl-20 amino-14β pregnane-5β triol-3β,12β,20β que l'on peut purifier par cristallisation.

Le produit ci-dessus est dissous dans 100 ml de chlorure de méthylène à 0° C, puis on ajoute 3,5 ml de triéthylamine et 3,1 ml d'anhydride trifluoroacétique. On maintient le mélange réactionnel sous agitation pendant environ 20 min puis on élimine le solvant par évaporation.

Le résidu obtenu est purifié par redissolution dans le chlorure de méthylène, puis lavage par une solution de bicarbonate de sodium suivant les techniques usuelles. Après dissolution dans le méthanol et passage sur une colonne de résine IR 45, on obtient 4,6 g de O-acétyl-20 trifluoroacétamido-14β pregnane-5β triol-3β,12β,20β.

A une solution de 3 g du dérivé obtenu comme indiqué ci-dessus et 2,7 g de bromure de tri-O-acétyl-2,3,4 L-rhamnosyle dans 160 ml d'acétonitrile, on ajoute 1,9 g de cyanure mercurique. Après 1 h d'agitation à température ambiante, on dilue par du chlorure de méthylène et on extrait en lavant par une solution saturée de carbonate de sodium. Après chromatographie sur silice Merck H60 en utilisant comme éluant un mélange chlorure de méthylène/méthanol (98,5/1,5), on obtient 3 g de O-(tri-O-acétyl-2,3,4 α-L-rhamnopyrannosyl)-3 O-acétyl-20 trifluoroacétamido-14β pregnane-5β triol-3β,12β,20β.

Le produit ainsi obtenu est dissous dans 60 ml de méthanol et additionné de 6 ml de soude 10N. On chauffe à reflux pendant 6 h, puis on dilue à l'eau et on extrait par du chlorure de méthylène. On obtient ainsi 1 g de O-(α-L-rhamnopyrannosyl)-3 amino-14β pregnane-5β triol-3β,12β,20β brut, que l'on purifie par chromatographie sur silice Merck H60 en utilisant comme éluant un mélange chlorure de méthylène/méthanol/ammoniaque (80/20/2).

On prépare le chlorhydrate correspondant par action de l'acide chlorhydrique concentré en solution méthanolique. Le produit formé est filtré, dissous dans l'isopropanol et précipité à l'éther.

Point de fusion F = 260° C

Spectre de RMN ($CDCl_3/CD_3OD$ 4/1)

δ = 0,93 (s, Me-19) 1,06 (s, Me-18) 1,26 (d, j = 7, Me 21) 1,27 (d, j = 6, Me-6') 3,28 (m, H-12) 4,78 (m, H1') ppm.

*Exemple 11:*

*Di-O-(α-L-rhamnopyrannosyl)-3,12 amino-14β pregnane-5β triol-3β,12β,20β*

A une solution de 3 g de O-acétyl-20 trifluoroacétamido-14β pregnane-5β triol-3β,12β,20β obtenu comme indiqué dans l'exemple 10, et 4,4 g de bromure de tri-O-acétyl-2,3,4 L-rhamnosyle dans 160 ml d'acétonitrile, on ajoute 3,2 g de cyanure mercurique.

On laisse réagir pendant 1 h sous agitation à température ambiante, on dilue par du chlorure de méthylène et on extrait en lavant par une solution saturée de carbonate de sodium. Le produit obtenu est dissous dans 100 ml de méthanol, et on ajoute 10 ml de soude 10N. On chauffe à reflux pendant 6 h environ, on évapore le solvant et on ajoute 50 ml d'eau. Il se forme un précipité qui est filtré et repris dans un mélange de chlorure de méthylène et de méthanol (90/10).

Après extraction, séchage et évaporation suivant les techniques usuelles, on obtient 3,1 g du produit recherché qui est purifié par cristallisation dans l'éthanol absolu.

Point de fusion F = 240° C

Spectre IR (Nujol) η = 3440, 3370, 3260, 1620, 1590 $cm^{-1}$.

Les expérimentations réalisées sur les dérivés d'amino-14 stéroïdes représentés par la formule générale (I) ont fait apparaître notamment une activité inotrope positive ainsi que des propriétés antiarythmiques supraventriculaires.

Plus particulièrement, les dérivés conformes à la présente invention présentent une activité inotrope positive supérieure ou égale à celle des composés de référence bien connus tels que la ouabaïne et la digoxine.

L'activité inotrope a été vérifiée sur l'oreillette isolée de cobaye dans les conditions expérimentales usuelles, en enregistrant l'amplitude des contractions pour diverses doses administrées, par rapport aux valeurs contrôles.

Le tableau 1 ci-après indique l'augmentation de la force de contraction en fonction de la concentration pour la digoxine (produit de comparaison) et pour les produits de l'invention décrits dans les exemples 6 et 7.

*Tableau 1*

*Amplitude des contractions par rapport aux valeurs contrôles*

| Composé | Concentration (moles/l) | | | | |
|---|---|---|---|---|---|
| | $2\times10^{-7}$ | $10^{-6}$ | $2\times10^{-6}$ | $6\times10^{-6}$ | $10^{-5}$ |
| Digoxine | +38% | +100% | +114% | +128% | +128% |
| Ex. n° 6 | — | + 43% | + 63% | +143% | +148% |
| Ex. n° 7 | +50% | + 97% | +178% | +233% | +206% |

Ces résultats montrent que les produits conformes à l'invention provoquent des augmentations de la force de contraction nettement supérieures à celles provoquées par la digoxine, pour certaines concentrations.

Par ailleurs, les dérivés d'amino-14 stéroïdes suivant l'invention présentent une capacité d'inhibition de l'ATPase membranaire moléculairement supérieure ou égale à celle de la digoxine ou de la ouabaïne prises comme références.

Ainsi par exemple, lorsque l'on compare les dérivés de l'invention décrits dans les exemples 2, 6 et 7, aux produits de comparaison constitués par la ouabaïne et la digoxine, on obtient les résultats exposés au tableau 2 ci-après.

Dans ce tableau, la valeur de DE50 est la dose provoquant 50% d'inhibition de l'ATPase membranaire.

*Tableau 2*

| | DE50 |
|---|---|
| Ouabaïne | $3{,}6\times10^{-9}$M |
| Digoxine | $5{,}3\times10^{-9}$M |
| Ex. n° 2 | $1{,}3\times10^{-10}$M |
| Ex. n° 6 | $1{,}8\times10^{-10}$M |
| Ex. n° 7 | $7{,}0\times10^{-11}$M |

Les dérivés conformes à l'invention se distinguent des substances connues de comparaison de la série des digitaliques, telles que la digoxine et la ouabaïne, par une modification avantageuse de leur activité toxique.

Par exemple, le dérivé de l'invention décrit dans l'exemple 6 permet d'atteindre, chez le chien anesthésié, sans signe toxique, un accroissement de la contractilité cardiaque de 150%, alors que, dans les mêmes conditions expérimentales, les signes toxiques apparaissent, pour la digoxine, lorsque la contractilité cardiaque a augmenté d'environ 50%. De plus, la toxicité des dérivés conformes à l'invention est réversible, c'est-à-dire que l'arrêt de leur administration suffit à faire disparaître les troubles observés à l'électrocardiogramme.

Ces résultats montrent que les dérivés suivant l'invention peuvent être utilisés en thérapeutique humaine et vétérinaire, comme médicaments notamment pour le traitement de l'insuffisance cardiaque.

Les dérivés de formule générale (I) et leurs sels pharmaceutiquement acceptables peuvent être administrés sous les formes usuelles, le principe actif étant dilué dans un support pharmaceutiquement acceptable convenablement choisi, par exemple sous forme de comprimé, gélule, dragée, suppositoire, soluté injectable ou sirop.

A titre d'exemple, les comprimés peuvent être préparés en mélangeant le dérivé de formule générale (I) ou un de ses sels, avec un ou plusieurs diluants solides tels que le lactose, le mannitol, l'amidon, la polyvinylpyrrolidone, le stéarate de magnésium, le talc, etc. Le cas échéant, les comprimés peuvent comporter plusieurs couches superposées autour d'un noyau, suivant les techniques usuelles, pour assurer une libération progressive ou un effet retardé du principe actif. L'enrobage peut par exemple être constitué d'une ou de plusieurs couches d'acétate de polyvinyle, de carboxyméthylcellulose ou d'acétophtalate de cellulose.

On a préparé des comprimés répondant aux formules ci-dessous:

Comprimé A:

O-(α-L-rhamnopyrannosyl)-3 amino-14β pregnane-5β triol-3β,12β,20β (chlorhydrate) 0,2 mg
excipient q.s.p. 100 mg
(excipient: amidon, talc, lactose, stéarate de magnésium).

Comprimé B:

(α-L-rhamnopyrannosyl)-3 amino-14β pregnane-5β diol-3β,20β 3 mg
excipient q.s.p. 100 mg
(excipient: amidon, talc, lactose, stéarate de magnésium).

On peut également administrer le dérivé suivant l'invention sous forme d'un sirop ou d'un soluté buvable obtenu en dissolvant le dérivé de formule (I) ou un de ses sels pharmaceutiquement acceptables, dans de l'eau ou du glycérol, par exemple, et en ajoutant le cas échéant un additif usuel tel qu'un édulcorant et un antioxydant.

Des solutions injectables peuvent être préparées suivant les techniques bien connues et sont constituées par exemple par un soluté contenant un dérivé de formule (I) ou un de ses sels pharmaceutiquement acceptables, dissous dans de l'eau bidistillée, une solution hydroalcoolique, du propylèneglycol, etc., ou un mélange de ces solvants. Le cas échéant, un additif approprié tel qu'un conservateur peut être ajouté.

Les doses administrées sont déterminées par le médecin en fonction du mode d'administration choisi, le niveau de l'affection traitée, la durée du traitement, etc. A titre d'exemple, dans le cas de l'administration orale chez l'homme, les doses peuvent être comprises entre 0,05 et 2 mg/kg, dans le cas d'un dérivé tel que celui des exemples 2, 6 ou 7. Le cas échéant, les doses doivent être plus faibles, et par exemple dans le cas du dérivé de l'exemple 10, elles sont de préférence comprises entre 0,001 et 0,5 mg/kg.

**Revendications** pour les Etats contractants BE, CH, LI, DE, FR, GB, IT, NL

1. Dérivés d'amino-14 stéroïdes représentés par la formule générale (I):

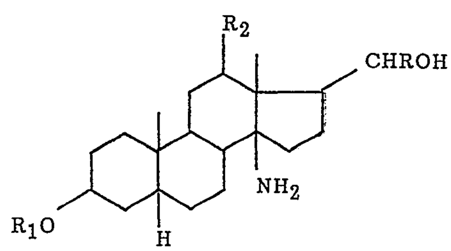

(I)

dans laquelle R représente un atome d'hydrogène ou un groupe alkyle inférieur de 1 à 4 atomes de carbone, $R_1$ représente un reste de sucre, substitué le cas échéant, et $R_2$ représente un atome d'hydrogène, un groupe hydroxyle ou un groupe $-OR_3$,

$R_3$ étant un reste de sucre, substitué le cas échéant, ainsi que leurs sels d'acides.

2. Dérivés d'amino-14 stéroïdes selon la revendication 1, caractérisé en ce que R représente un atome d'hydrogène ou un groupe méthyle.

3. Dérivés d'amino-14 stéroïdes selon l'une des revendications 1 ou 2, caractérisés en ce que $R_1$ est un reste de monosaccharide substitué ou non substitué, et $R_2$ est un atome d'hydrogène ou un groupe hydroxyle.

4. Dérivés d'amino-14 stéroïdes selon la revendication 3, caractérisés en ce que $R_1$ représente un reste de pentose ou d'hexose, modifié ou substitué le cas échéant.

5. Dérivés d'amino-14 stéroïdes selon la revendication 4, caractérisés en ce que $R_1$ représente un reste de glucose, de rhamnose, de galactose, de fucose ou de digitoxose, substitué le cas échéant.

6. Dérivés d'amino-14 stéroïdes selon la revendication 1, caractérisés en ce qu'ils sont choisis parmi le O-(α-L-rhamnopyrannosyl)-3 amino-14β nor-21 pregnane-5β diol-3β,20, le O-(α-L-rhamnopyrannosyl)-3 amino-14β pregnane-5β diol-3β,20α et son isomère 20β, le O-(β-D-digitoxosyl)-3 amino-14β pregnane-5β diol-3β,20α, le O-(amino-4 tridésoxy-2,3,6 α-L-lyxohexopyrannosyl)-3 amino-14β pregnane-5β diol-3β,20α, le O-(α-L-rhamnopyrannosyl)-3 amino-14β nor-21 pregnane-5β triol-3β,12β,20, le O-(α-L-rhamnopyrannosyl)-3 amino-14β pregnane-5β triol-3β,12β,20β, et son isomère 20α, le O-(β-D-digitoxosyl)-3 amino-14β pregnane-5β triol-3β,12β,20α, et le di-O-(α-L-rhamnopyrannosyl)-3,12 amino-14β pregnane-5β triol-3β,12β,20β.

7. Procédé de préparation de dérivés d'amino-14 stéroïdes selon la revendication 1, caractérisé en ce qu'on effectue une réaction de couplage entre un amino-14 stéroïde de formule générale (II):

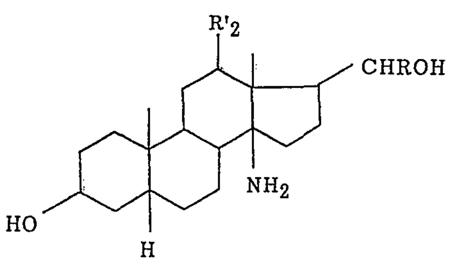

(II)

dans laquelle R est un atome d'hydrogène ou un groupe alkyle inférieur, et $R'_2$ est un atome d'hydrogène ou un groupe hydroxyle, après avoir préalablement protégé le groupe $-NH_2$ en position 14 et le groupe $-OH$ en position 20, avec un sucre activé de formule $R_1-X$, où $R_1$ est un reste sucré et X est un atome d'halogène ou un groupe acétyle, puis on élimine, le cas échéant, les groupements protecteurs.

8. Procédé selon la revendication 7, caractérisé en ce que le dérivé sucré de formule $R_1-X$ est ajouté en excès.

9. Procédé selon la revendication 7, caractérisé en ce que le groupe $-NH_2$ en position 14 est préalablement protégé par transformation en groupe trifluoroacétamido par action de l'anhydride trifluoroacétique en présence de triéthylamine, ou en groupe formamido par action de l'anhydride acétique dans l'acide formique, et le groupe $-OH$ en position 20 est préalablement protégé par acétylation par l'anhydride acétique dans la pyridine.

10. Compositions pharmaceutiques, caractérisées en ce qu'elles contiennent un dérivé d'amino-14 stéroïde selon l'une des revendications 1 à 6, comme principe actif associé à un ou plusieurs excipients pharmaceutiquement acceptables.

**Revendications** pour l'Etat contractant: AT

1. Procédé de préparation de dérivés d'amino-14 stéroïdes représentés par la formule générale (I):

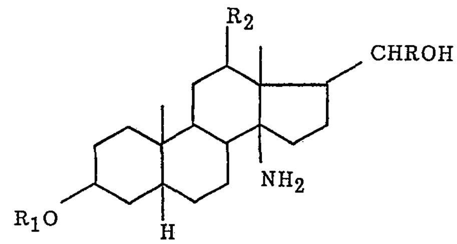

(I)

dans laquelle R représente un atome d'hydrogène ou un groupe alkyle inférieur de 1 à 4 atomes de carbone, $R_1$ représente un reste de sucre, substitué le cas échéant, et $R_2$ représente un atome d'hydrogène, un groupe hydroxyle ou un groupe $-OR_3$, $R_3$ étant un reste de sucre, substitué le cas échéant, caractérisé en ce qu'on effectue une réaction de couplage entre un amino-14 stéroïde de formule générale (II):

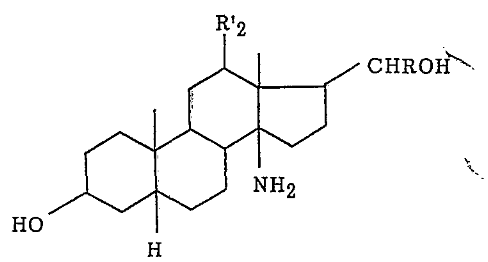

(II)

dans laquelle R est un atome d'hydrogène ou un groupe alkyle inférieur, et $R'_2$ est un atome d'hydrogène ou un groupe hydroxyle, après avoir préalablement protégé le groupe $-NH_2$ en position 14 et le groupe $-OH$ en position 20, avec un sucre activé de formule $R_1-X$, où $R_1$ est un reste sucré et X est un atome d'halogène ou un groupe acétyle, puis on élimine, le cas échéant, les groupements protecteurs.

2. Procédé selon la revendication 1, caractérisé en ce que le dérivé sucré de formule $R_1-X$ est ajouté en excès.

3. Procédé selon la revendication 1, caractérisé en ce que le groupe $-NH_2$ en position 14 est préalablement protégé par transformation en groupe trifluoroacétamido par action de l'anhydride trifluoroacétique en présence de triéthylamine, ou en groupe formamido par action de l'anhydride acétique dans l'acide formique, et le groupe $-OH$ en

position 20 est préalablement protégé par acétylation par l'anhydride acétique dans la pyridine.

**Claims** for the Contracting States BE, CH, LI, DE, FR, GB, IT, NL, SE

1. 14-amino steroid derivatives represented by general Formula (I):

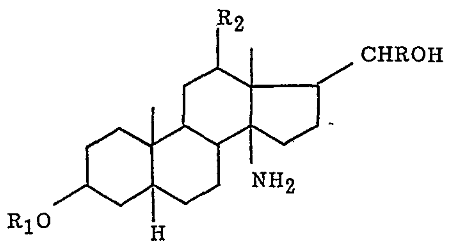

(I)

wherein R represents a hydrogen atom or a lower alkyl group having 1 to 4 carbon atoms, $R_1$ represents a substituted or unsubstituted sugar residue, and $R_2$ represents a hydrogen atom, a hydroxyl group or an $-OR_3$ group, wherein $R_3$ is a substituted or unsubstituted sugar residue, and acid salts thereof.

2. The 14-amino steroid derivatives of Claim 1, characterized in that R represents a hydrogen atom or a methyl group.

3. The 14-amino steroid derivatives of any one of Claims 1 and 2, characterized in that $R_1$ is a substituted or unsubstituted monosaccharide residue, and $R_2$ is a hydrogen atom or a hydroxyl group.

4. The 14-amino steroid derivatives of Claim 3, characterized in that $R_1$ represents a pentose or hexose residue, possibly modified or substituted.

5. The 14-amino steroid derivatives of Claim 4, characterized in that $R_1$ represents a substituted or unsubstituted glucose residue, rhamnose residue, galactose residue, fucose residue or digitoxose residue.

6. The 14-amino steroid derivatives of Claim 1, characterized in that said derivatives are selected from 3-O-(α-L-rhamnopyranosyl) 14β-amino 21-nor 5β-pregnane 3β,20-diol, 3-O-(α-L-rhamnopyranosyl) 14β-amino 5β-pregnane 3β,20α-diol and its 20β isomer, 3-O-(β-D-digitoxosyl) 14β-amino 5β-pregnane 3β,20α-diol, 3-O-(4-amino 2,3,6-trideoxy α-L-lyxohexopyranosyl) 14β-amino 5β-pregnane 3β,20α-diol, 3-O-(α-L-rhamnopyranosyl) 14β-amino 21-nor 5β-pregnane 3β,12β,20-triol, 3-O-(α-L-rhamnopyranosyl) 14β-amino 5-β-pregnane 3β,12β,20β-triol and its 20α isomer, 3-O-(β-D-digitoxosyl) 14β-amino 5β-pregnane 3β,12β,20α-triol and 3,12-di-O-(α-L-rhamnopyranosyl) 14β-amino 5β-pregnane 3β,12β,20β-triol.

7. A process for the preparation of the 14-amino steroid derivatives of Claim 1, characterized in that it comprises coupling the 14-amino steroid of general Formula (II):

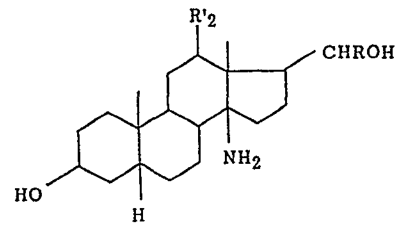

(II)

wherein R is a hydrogen atom or a lower alkyl group, and $R'_2$ is a hydrogen atom or a hydroxyl group, after preliminary protection of the $-NH_2$ group at the 14-position and the $-OH$ group at the 20-position, with an activated sugar of the formula $R_1-X$, wherein $R_1$ is a sugar residue and X is a halogen atom or an acetylated group; and, if, desired, removing the protective groups.

8. The process of Claim 7, characterized in that the sugar derivative of the formula $R_1-X$ is added in excess.

9. The process of Claim 7, characterized that the $-NH_2$ group at the 14-position is protected by transformation into a trifluoroacetamido group by the action of trifluoroacetic anhydride in the presence of triethylamine, or into a formamido group by the action of acetic anhydride in formic acid; and the $-OH$ group at the 20-position is protected by acetylation by acetic anhydride in pyridine.

10. Pharmaceutical compositions characterized in that they comprise a 14-amino steroid derivative of any one of Claims 1 to 6 as an active ingredient combined with one or several pharmaceutically acceptable carriers.

**Claims** for the Contracting State: AT

1. A process for the preparation of 14-amino steroid derivatives represented by general Formula (I):

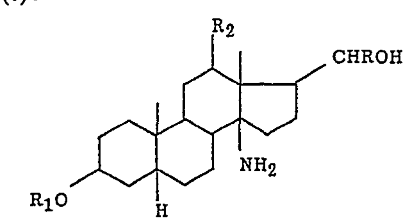

(I)

wherein R represents a hydrogen atom or a lower alkyl group having 1 to 4 carbon atoms, $R_1$ represents a substituted or unsubstituted sugar residue, and $R_2$ represents a hydrogen atom, a hydroxyl group or an $-OR_3$ group, wherein $R_3$ is a substituted or unsubstituted sugar residue characterized in that it comprises coupling the 14-amino steroid of general Formula (II):

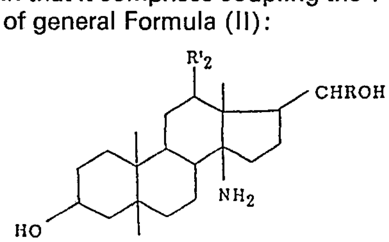

(II)

wherein R is a hydrogen atom or a lower alkyl group, and $R'_2$ is a hydrogen atom or a hydroxyl group, after preliminary protection of the $-NH_2$ group at the 14-position and the $-OH$ group at the 20-position, with an activated sugar of the formula $R_1-X$, wherein $R_1$ is a sugar residue and X is a halogen atom or an acetylated group; and, if desired, removing the protective groups.

2. The process of Claim 1, characterized in that the sugar derivative of the formula $R_1-X$ is added in excess.

3. The process of Claim 1, characterized that the $-NH_2$ group at the 14-position is protected by transformation into a trifluoroacetamido group by the action of trifluoroacetic anhydride in the presence of triethylamine, or into a formamido group by the action of acetic anhydride in formic acid; and the $-OH$ group at the 20-position is protected by acetylation by acetic anhydride in pyridine.

**Patentansprüche** für die Vertragstaaten BE, CH, LI, DE, FR, GB, IT, NL, SE

1. Derivate von 14-Amino-Steroiden der allgemeinen Formel (I):

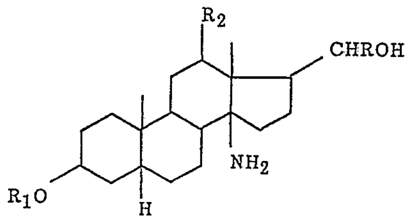

(I)

worin R ein Wasserstoffatom oder eine niedere Alkylgruppe mit 1 bis 4 Kohlenstoffatomen, $R_1$ einen ggf. substituierten Zuckerrest und $R_2$ ein Wasserstoffatom, eine Hydroxygruppe oder eine $-OR_3$-Gruppe, worin $R_3$ ein ggf. substituierter Zuckerrest ist, bedeutet, sowie ihre Salze mit Säuren.

2. Derivate von 14-Amino-Steroiden nach Anspruch 1, dadurch gekennzeichnet, dass R ein Wasserstoffatom oder eine Methylgruppe bedeutet.

3. Derivate von 14-Amino-Steroiden nach einem der Ansprüche 1 oder 2, dadurch gekennzeichnet, dass $R_1$ ein substituierter oder nicht substituierter Monosaccharidrest und $R_2$ ein Wasserstoffatom oder eine Hydroxygruppe ist.

4. Derivate von 14-Amino-Steroiden nach Anspruch 3, dadurch gekennzeichnet, dass $R_1$ einen ggf. modifizierten oder substituierten Pentose- oder Hexoserest bedeutet.

5. Derivate von 14-Amino-Steroiden nach Anspruch 4, dadurch gekennzeichnet, dass $R_1$ einen ggf. substituierten Glucose-, Rhamnose-, Galactose-, Fucose- oder Digitoxoserest bedeutet.

6. Derivate von 14-Amino-Steroiden nach Anspruch 1, dadurch gekennzeichnet, dass sie aus O-(α-L-Rhamnopyranosyl)-3-amino-14β-nor-21-pregnan-5β-diol-3β,20, O-(α-L-Rhamnopyranosyl)-3-amino-14β-pregnan-5β-diol-3β,20α und seinem 20β-Isomer, O-(β-D-Digitoxosyl)-3-amino-14β-pregnan-5β-diol-3β,20α, O-(amino-4-tridesoxy-2,3,6-α-L-lyxohexopyranosyl)-3-amino-14β-pregnan-5β-diol-3β,20α, O-(α-L-Rhamnopyranosyl)-3-amino-14β-nor-21-)pregnan-5β-triol-3β,12β,20, O-(α-L-Rhamnopyranosyl)-3-amino-14β-pregnan-5β-triol-)3β,12β,20β und seinem 20α-Isomer, O-(β-D-Digitoxosyl)-3-amino-14β-pregnan-5β-triol-3β,12β,20α und Di-O-(α-L-Rhamnopyranosyl)-3,12-amino-14β-pregnan-5β-triol-3β,12β,20β ausgewählt sind.

7. Verfahren zur Herstellung von Derivaten von 14-Amino-Steroiden nach Anspruch 1, dadurch gekennzeichnet, dass man die Kopplungsreaktion zwischen einem 14-Amino-Steroid der allgemeinen Formel (II):

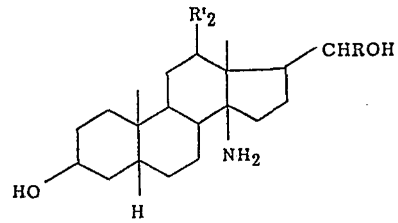

(II)

worin R ein Wasserstoffatom oder eine niedere Alkylgruppe ist und $R_2'$ ein Wasserstoffatom oder eine Hydroxygruppe ist, nachdem man vorher die $-NH_2$-Gruppe in 14-Stellung und die $-OH$-Gruppe in 20-Stellung geschützt hat, und einem aktivierten Zucker der Formel $R_1-X$, worin $R_1$ ein Zuckerrest und X ein Halogenatom oder eine Acetylgruppe ist, durchführt und dann ggf. die Schutzgruppen abspaltet.

8. Verfahren nach Anspruch 7, dadurch gekennzeichnet, dass das Zuckerderivat der Formel $R_1-X$ im Überschuss zugegeben wird.

9. Verfahren nach Anspruch 7, dadurch gekennzeichnet, dass die $-NH_2$-Gruppe in 14-Stellung vorher durch Umwandlung in eine Trifluoracetamidgruppe durch Einwirken von Trifluoressigsäureanhydrid in Gegenwart von Triethylamin oder in eine Formamidgruppe durch Einwirken von Essigsäureanhydrid in Ameisensäure geschützt wird und die $-OH$-Gruppe in 20-Stellung vorher durch Acetylierung mit Essigsäureanhydrid in Pyridin geschützt wird.

10. Pharmazeutische Zusammensetzungen, dadurch gekennzeichnet, dass sie ein Derivat von 14-Amino-Steroid nach einem der Ansprüche 1 bis 6 als wirksames Prinzip zusammen mit einem oder mehreren pharmazeutisch annehmbaren Exzipienten enthalten.

**Patentansprüche** für den Vertragstaat: AT

1. Verfahren zur Herstellung von Derivaten von 14-Amino-Steroiden der allgemeinen Formel (I):

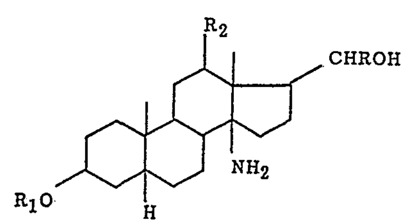

(I)

worin R ein Wasserstoffatom oder eine niedere Alkylgruppe mit 1 bis 4 Kohlenstoffatomen, $R_1$ einen ggf. substituierten Zuckerrest und $R_2$ ein Wasserstoffatom, eine Hydroxygruppe oder eine $-OR_3$-Gruppe, worin $R_3$ ein ggf. substituierter Zuckerrest ist, bedeutet, dadurch gekennzeichnet, dass man eine Kopplungsreaktion zwischen einem 14-Amino-Steroid der allgemeinen Formel (II):

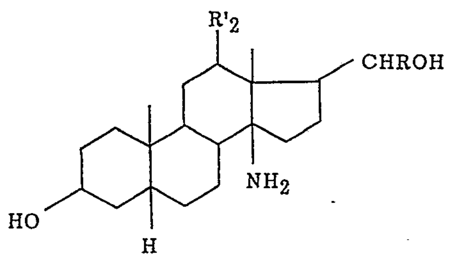

(II)

worin R ein Wasserstoffatom oder eine niedere Alkylgruppe ist und $R_2'$ ein Wasserstoffatom oder eine Hydroxygruppe ist, nachdem man vorher die $-NH_2$-Gruppe in 14-Stellung und die $-OH$-Gruppe in 20-Stellung geschützt hat, mit einem aktivierten Zucker der Formel $R_1-X$, worin $R_1$ ein Zuckerrest und X ein Halogenatom oder eine Acetlygruppe ist, durchführt und dann ggf. die Schutzgruppen abspaltet.

2. Verfahren nach Anspruch 1, dadurch gekennzeichnet, dass das Zuckerderivat der Formel $R_1-X$ im Überschuss zugegeben wird.

3. Verfahren nach Anspruch 1, dadurch gekennzeichnet, dass die $-NH_2$-Gruppe in 14-Stellung vorher durch Umwandlung in eine Trifluoracetamidgruppe durch Einwirken von Trifluoressigsäureanhydrid in Gegenwart von Triethylamin oder in eine Formamidgruppe durch Einwirken von Essigsäureanhydrid in Ameisensäure und die $-OH$-Gruppe in 20-Stellung vorher durch Acetylierung mit Essigsäureanhydrid in Pyridin geschützt wird.